# EUROPÄISCHE PATENTSCHRIFT

(11) **EP 3 221 007 B1**
(45) Veröffentlichungstag und Bekanntmachung des Hinweises auf die Patenterteilung: **06.05.2020**
(21) Anmeldenummer: 15778335.8
(22) Anmeldetag: 12.10.2015
(51) Int. Cl.: A61Q 11/00, A61K 8/21, A61K 8/73, A61K 8/81, A61K 8/44

(54) **MUND- UND ZAHNPFLEGE- UND -REINIGUNGSMITTEL FÜR ÜBEREMPFINDLICHE ZÄHNE**
ORAL AND DENTAL HYGIENE AND CLEANING AGENTS FOR HYPERSENSITIVE TEETH
PRODUITS DE NETTOYAGE ET D'HYGIÈNE BUCCO-DENTAIRE POUR DENTS HYPERSENSIBLES

(30) Priorität: 18.11.2014 DE 102014223525
(43) Veröffentlichungstag der Anmeldung: 27.09.2017
(73) Patentinhaber: Henkel AG & Co. KGaA, 40589 Düsseldorf (DE)
(72) Erfinder: MIEHLICH, Kristin, 42119 Wuppertal (DE); ARIANS, Daniela, 45239 Essen (DE); EVENING, Jennifer, 40472 Düsseldorf (DE)
(86) Internationale Anmeldenummer: PCT/EP2015/073533
(87) Internationale Veröffentlichungsnummer: WO 2016/078831

(56) Entgegenhaltungen:
- EP-A2- 0 867 174
- DATABASE GNPD [Online] MINTEL; 1. März 2014 (2014-03-01), "Fresh Mint Toothpaste", XP002751824, Database accession no. 2349246

## Beschreibung

Die Erfindung betrifft Mund- und Zahnpflege- und -reinigungsmittel für überempfindliche Zähne mit einem amphoteren Tensid und einem Polymer.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Große Teile der erwachsenen Bevölkerung besitzen überempfindliche Zähne, Die Überempfindlichkeit der Zähne wird durch die freiliegenden Öffnungen der Dentintubuli verursacht, die externe Reize an die Pulpa, die weiche, blutgefässreiche Gewebemasse im Zahn, weiterleiten. Freiliegendes Dentin wird hauptsächlich durch Zahnfleischrückgang aufgrund von Rezession, Abrasion, Zahnfleischverletzungen oder Parodontalbehandlungen verursacht. Liegt Dentin frei, stellen die Dentintubuli eine direkte Verbindung zwischen der Mundhöhle und der Pulpa dar. Externe Reize wie mechanischer Druck, Temperaturschwankungen (heiß - kalt) sowie chemische Reize (süß - sauer) werden über die Dentintubuli an die Pulpa weitergeleitet und aktivieren den Nerv. Es folgt ein kurzer, scharfer Schmerz.

Im Stand der Technik sind Mittel und Verfahren zur Verringerung der Überempfindlichkeit von Zähnen bekannt, bei denen beispielsweise die Dentintubuli mit (geladenen) Polymerverbindungen verschlossen werden. Aus der WO 2001/39737 A1 sind beispielsweise Mundpflegemittel bekannt, die zur Verringerung der Überempfindlichkeit der Zähne eine Kombination von einem Aminhydrofluorid und einem Additionssalz eines Vinylpyrrolidon/2-Dimethylaminoethylmethacrylat-Copolymers mit einer pharmazeutisch verträglichen Säure oder eine Kombination von einem Aminhydrofluorid und einem Polyurethan enthalten.

Aus der EP 0867174 A2 ist eine Zahncremezusammensetzung bekannt, die Polyquaternium-10 und Natriumfluorid enthält.

Es besteht allerdings weiterhin ein Bedarf, die Überempfindlichkeit von Zähnen bei deren Pflege-und/oder Reinigung zu verringern und Mund- und Zahnpflege- und -reinigungsmittel diesbezüglich zu verbessern.

Es wurde nun gefunden, dass sich während der Behandlung von Zähnen durch den Einsatz einer Kombination aus einem amphoteren Tensid, einer Polyquaternium-Verbindung und Fluorid freiliegende Dentintubuli verschliessen und eine schmerzhafte Reizweiterleitung verhindern lassen.

Gegenstand der vorliegenden Erfindung ist in einer ersten Ausführungsform ein Mund- und Zahnpflege- und -reinigungsmittel, enthaltend - bezogen auf sein Gewicht -
a) mindestens eine Verbindung, die unter die INCI-Bezeichnung Polyquaternium fällt, ausgewählt aus der Gruppe bestehend aus Verbindungen, die unter die INCI-Bezeichnungen Polyquaternium-7 und Polyquaternium-10 fallen, und deren Mischungen,
b) 0,01 bis 10 Gew.-% amphotere(s) Tensid(e) , umfassend eine unter der INCI-Bezeichnung Cocamidopropyl Betaine bekannte Verbindung und/oder eine unter der INCI-Bezeichnung Disodium Cocoamphodiacetate bekannte Verbindung und
c) 500 bis 1600 ppm Fluorid.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes, Mund- und Zahnspülungen sowie Mund- und Zahngele. Bevorzugt geeignet sind Zahnpasten und flüssige Zahnreinigungsmittel. Hierzu können die Mund- und Zahnpflege- und -reinigungsmittel zum Beispiel in Form von Zahnpasten, flüssigen Zahncremes, Zahnpulvern, Mundwässern oder gegebenenfalls auch als Kaumasse, zum Beispiel als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden. Eine weitere besonders bevorzugte Ausführungsform der vorliegenden Erfindung sind Mundspüllösungen und Mundwasser, die zum Ausspülen der Mundhöhle verwendet werden.

Als ersten wesentlichen Inhaltsstoff enthalten die Mittel mindestens eine Verbindung, die unter die INCI-Bezeichnung Polyquaternium fällt und ausgewählt ist aus der Gruppe bestehend aus Verbindungen, die unter die INCI-Bezeichnungen Polyquaternium-7 und Polyquäternium-10 fallen, und deren Mischungen. Polyquaternium ist nach der INCI-Nomenklatur, Regel 23, eine Bezeichnungen für komplexe quarternäre Ammonium-Verbindungen. Die verschiedenen Vertreter dieser Gruppe werden durch Ziffern unterschieden.

Polyquaternium-7 (CAS-Nummer: 26590-05-6) ist definiert als polymeres quaternäres Ammoniumsalz hergestellt aus Acrylamid- und Dimethyldiallylammoniumchlorid-Monomeren.

Merquat 550 und Merquat S (ex Lubrizol), Conditioneze 7 MP (ex ISP) oder Galsilk 700 (Galaxy Surfactants) sind kommerziell erhältliche Formulierungen mit Polyquaternium-7.

Polyquaternium-10 (CAS-Nummern: 53568-66-4, 54351-50-7, 55353-19-0, 68610-92-4 und 81859-24-7) ist definiert als ein polymeres quaternäres Ammoniumsalz, welches aus der Umsetzung von Hydroxyethylcellulose mit einem Trimethylamrnonium-substitutierten Epoxid resultiert.

Die kationischen Cellulosen können beispielsweise unter den Bezeichnungen Ucare® Polymer JR 30 oder Ucare® Polymer JR 400 (ex Dow), Celquat SC 230 M oder Celquat SC 240 C (ex Akzo Nobel) sowie Polyquta 400 KCH (ex KCl) kommerziell erworben werden.

Im Folgenden werden die Bezeichnungen "Verbindung, die unter die INCI-Bezeichnung Polyquaternium fällt" und "Polyquaternium-Verbindung" sowie die Bezeichnungen "Verbindungen, die unter die INCI-Bezeichnung Polyquaternium fallen" und "Polyquaternium-Verbindungen" synonym verwendet.

Ohne an diese Theorie gebunden sein zu wollen, wird vermutet, dass die eingesetzte Polyquaternium-Verbindung oder die eingesetzte Mischung aus Polyquaternium-Verbindungen einen sehr dünnen Film auf der Oberfläche der Zähne ausbildet, der die Dentintubuli verschließt.

Bevorzugte Mund- und Zahnpflege- und -reinigungsmittel zeichnen sich dadurch aus, dass sie - bezogen auf ihr Gewicht - 0,001 - 5 Gew.-%, vorzugsweise 0,01 - 4 Gew.-%, besonders bevorzugt 0,05 - 3 Gew.-%, außerordentlich bevorzugt 0,1 - 2 Gew.-% und insbesondere 0,5 bis 1,5 Gew.-% an Verbindung(en), die unter die INCI-Bezeichnung Polyquaterniumfallen und ausgewählt ist/sind aus der Gruppe bestehend aus Verbindungen, die unter die INCI-Bezeichnungen Polyquaternium-7 und Polyquaternium-10 fallen, und deren Mischungen, enthalten.

Als zweiten wesentlichen Inhaltsstoff enthalten die Mittel 0,01 bis 10 Gew.-% amphotere(s) Tensid(e), umfassend eine unter der INCI-Bezeichnung Cocamidopropyl Betaine bekannte Verbindung und/oder eine unter der INCI-Bezeichnung Disodium Cocoamphodiacetate bekannte Verbindung. Besonders bevorzugte Mund- und Zahnpflege- und -reinigungsmittel enthalten 0,1 bis 5 Gew,-%, vorzugsweise 0,5 bis 3 Gew und insbesondere 1 bis 2 Gew.-% .-% amphotere(s) Tensid(e), umfassend eine unter der INCI-Bezeichnung Cocamidopropyl Betaine bekannte Verbindung und/oder eine unter der INCI-Bezeichnung Disodium Cocoamphodiacetate bekannte Verbindung.

Es sind Mund- und Zahnpflege- und -reinigungsmittel bevorzugt, die - bezogen auf ihr Gewicht - 0,01 bis 4,0 Gew.-%, vorzugsweise 0,1 bis 3,0 Gew.-%, besonders bevorzugt 0,25 bis 2,5 Gew.-%, weiter bevorzugt 0,4 bis 2,0 Gew.-% und insbesondere 0,5 bis 1,5 Gew.-% Cocamidopropylbetain enthalten.

Als dritten wesentlichen Inhaltsstoff enthalten die Mittel Fluorid. Diese kann in Form anorganischer Fluoridsalze (Natriumfluorid, Zinn(II)fluorid, Natriummonofluorphosphat usw.) bereitgestellt werden, auch Aminfluoride wie Olaflur sind geeignet.

Überraschenderweise wurde gefunden, dass die Fluoriddeposition gesteigert werden kann, wenn oberhalb bestimmter Fluoridgehalte zusätzlich Polyquaternium-Verbindung(en) in den Mitteln enthalten sind. Die Mindestmenge an Fluorid beträgt dabei 500 ppm, unterhalb dieser Grenze macht sich der Einsatz von Polyquaternium-Verbindung(en) auf die Fluoriddeposition nicht bemerkbar.

Besonders bevorzugte Mund- und Zahnpflege- und -reinigungsmittel sind dadurch gekennzeichnet, dass sie 1225 bis 1575 ppm, vorzugsweise 1250 bis 1550 ppm, weiter bevorzugt 1275 bis 1525 ppm, noch weiter bevorzugt 1300 bis 1500 ppm, noch weiter bevorzugt 1325 bis 1475 ppm und insbesondere 1350 bis 1450 ppm Fluorid enthalten.

Wird Fluorid in Form von Natriumfluorid bereitgestellt, entspricht 1 Gew.-% Natriumfluorid ungefähr 4524 ppm Fluorid, so dass bevorzugte Mittel 0,27 bis 0,35 Gew.-%, vorzugsweise 0,28 bis 0,34 Gew.-%, weiter bevorzugt 0,29 bis 0,33 Gew.-% und insbesondere 0,30 bis 0,32 Gew.-% Natriumfluorid enthalten.

Die Mund- und Zahnpflege- und -reinigungsmittel enthalten weitere Inhaltsstoffe. Bevorzugt ist hierbei der Einsatz von sogenannten Feuchthaltemitteln, die bei Zahnpasten das Austrocknen verhindern und die Wirksamkeit der Kombination aus Polyquaternium-Verbindung(en), amphoterem Tensid/amphoteren Tensiden und Fluorid steigern. Bei sogenannten Flüssigzahncremes mit fließfähiger Rheologie dienen diese als Matrix und werden in höheren Mengen eingesetzt. Bei Mundwässern und Mundspülungen dienen diese Alkohole als Konsistenzregler und zusätzliche Süßungsmittel.

Hier sind Mund- und Zahnpflege- und -reinigungsmittel bevorzugt, die - bezogen auf ihr Gewicht - 0,5 bis 70 Gew.-%, vorzugsweise 0,75 bis 60 Gew.-%, besonders bevorzugt 1 bis 55 Gew.-% und insbesondere 2 bis 50 Gew.-% mindestens eines mehrwertigen Alkohols aus der Gruppe Sorbit und/oder Glycerin und/oder 1,2-Propylenglycol oder deren Mischungen enthalten.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1) : (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können zum Beispiel Xylit, Propylenglycole, Polyethylenglycole, insbesondere solche mit mittleren Molekulargewichten von 200-800 eingesetzt werden.

Als weiteren Inhaltsstoff können die Mittel weitere Tensid(e), insbesondere anionische Tenside, enthalten. Auch die weiteren Tenside werden vorzugsweise innerhalb enger Mengenbereiche eingesetzt, so dass bevorzugte Mund- und Zahnpflege- und -reinigungsmittel dadurch gekennzeichnet sind, dass sie 0,5 bis 5 Gew.-%, vorzugsweise 0,75 bis 4,5 Gew.-%, weiter bevorzugt 1 bis 4 Gew.-%, noch weiter bevorzugt 1,25 bis 3,5 Gew.-% und insbesondere 1,6 bis 2,5 Gew.-% Tensid(e) enthalten.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Ganz besonders bevorzugte Mittel enthalten Alkylsulfat(e) als anionisches Tensid. Hier sind besonders bevorzugte Mund- und Zahnpflege- und -reinigungsmittel dadurch gekennzeichnet, dass sie 0,1 bis 4,0 Gew.-%, vorzugsweise 0,75 bis 3,0 Gew.-%, besonders bevorzugt 1,0 bis 2,5 Gew.-%, weiter bevorzugt 1,25 bis 2,0 Gew.-% und insbesondere 1,5 bis 1,8 Gew.-% Natriumlaurylsulfat enthalten.

Ebenfalls ganz besonders bevorzugte Mittel enthalten Fettalkoholethersulfate als anionisches Tensid. Bevorzugte Fettalkoholethersulfate sind solche der Formel

R¹-O-(AO)ₙ-SO₃⁻ X⁺.

In dieser Formel steht R¹ für einen linearen oder verzweigten, substituierten oder unsubstituierten Alkylrest, vorzugsweise für einen linearen, unsubstituierten Alkylrest, besonders bevorzugt für einen Fettalkoholrest. Bevorzugte Reste R¹ sind ausgewählt aus Decyl-, Undecyl-, Dodecyl-, Tridecyl-, Tetradecyl, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste R¹ sind abgeleitet von C₁₂-C₁₈-Fettalkoholen, beispielsweise von Kokosfettalkohol, Talgfettalkohol, Lauryl-, Myristyl-, Cetyl- oder Stearylalkohol oder von C₁₀-C₂₀-Oxoalkoholen.

AO steht für eine Ethylenoxid- (EO) oder Propylenoxid- (PO) Gruppierung, vorzugsweise für eine Ethylenoxidgruppierung. Der Index n steht für eine ganze Zahl von 1 bis 50, vorzugsweise von 1 bis 20 und insbesondere von 2 bis 10. Ganz besonders bevorzugt steht n für die Zahlen 2, 3, 4, 5, 6, 7 oder 8. X steht für ein einwertiges Kation oder den n-ten Teil eines n-wertigen Kations, bevorzugt sind dabei die Alkalimetallionen und darunter Na⁺ oder K⁺, wobei Na⁺ äußerst bevorzugt ist. Weitere Kationen X+ können ausgewählt sein aus NH₄⁺, ½ Zn²⁺,½ Mg²⁺,½ Ca²⁺,½ Mn²⁺, und deren Mischungen.

Besonders bevorzugte Mund- und Zahnpflege- und -reinigungsmittel enthalten ein Fettalkoholethersulfat ausgewählt aus Fettalkoholethersulfaten der Formel 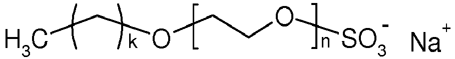 mit k = 11 bis 19, n = 2, 3, 4, 5, 6, 7 oder 8. Ganz besonders bevorzugte Vertreter sind C₁₂₋₁₄ Fettalkoholethersulfate mit 2 EO (k = 11-13, n = 2) und dabei insbesondere deren Na-Salze. Der angegebenen Ethoxylierungsgrad stellt einen statistischen Mittelwert dar, der für ein spezielles Produkt eine ganze oder eine gebrochene Zahl sein kann.

Hier sind besonders bevorzugte Mund- und Zahnpflege- und -reinigungsmittel dadurch gekennzeichnet, dass sie 0,1 bis 4,0 Gew.-%, vorzugsweise 0,75 bis 3,0 Gew.-%, besonders bevorzugt 1,0 bis 2,5 Gew.-%, weiter bevorzugt 1,25 bis 2,0 Gew.-% und insbesondere 1,5 bis 1,8 Gew.-% Natriumlaurylethersulfat mit 2 EO enthalten.

Zur Entfaltung der Reinigungsleistung und für eine "natürliche" Farbaufhellung von Zähnen können insbesondere die Zahnpflege- und -reinigungsmittel Poliermittel enthalten. Als Poliermittel eignen sich prinzipiell alle für Zahnpflege- und -reinigungsmittel bekannten Putzkörper, insbesondere solche, die keine Calciumionen enthalten. Bevorzugt geeignete Poliermittel-komponenten sind daher Kieselsäuren, Aluminiumhydroxid, Aluminiumoxid, Natriumaluminiumsilikate, organische Polymere oder Gemische solcher Putzkörper.

Calciumhaltige Polierkomponenten wie zum Beispiel Kreide, Calciumpyrophosphat, Dicalciumphosphatdihydrat können aber in Mengen bis zu 5 Gew.-% - bezogen auf das gesamte Mittel - enthalten sein.

Der Gesamtgehalt an Poliermitteln liegt vorzugsweise im Bereich von 5-50 Gew.-% des Zahnpflege- und -reinigungsmittels.

Besonders bevorzugt sind Zahnpasten und flüssige Zahnreinigungsmittel, die als Poliermittel Kieselsäuren enthalten. Geeignete Kieselsäuren sind zum Beispiel Gelkieselsäuren, Hydrogelkieselsäuren und Fällungskieselsäuren. Gelkieselsäuren werden durch Umsetzung von Natriumsilikatlösungen mit starken, wässrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die sogenannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident®12 DS (DEGUSSA) erhältlich.

Andere Fällungskieselsäuren dieser Art sind Sident® 8 (DEGUSSA) und Sorbosil® AC 39 (Crosfield Chemicals). Diese Kieselsäuren zeichnen sich durch eine geringere Verdickungswirkung und eine etwas höhere mittlere Teilchengröße von 8 -14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 -100 Pa.s aufweisen.

Zahnpasten, die eine deutlich höhere Viskosität von mehr als 100 Pa.s (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, sogenannte Verdickungskieselsäuren mit einer BET-Oberfläche von 150 -250 m²/g zu, zum Beispiel die Handelsprodukte Sipernat® 22 LS oder Sipernat® 320 DS.

Ebenso kann als Poliermittel auch Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich. Es ist jedoch bevorzugt, dass Aluminiumoxid als weiteres Poliermittel, das heißt in Kombination mit einem Poliermittel, in den Mitteln eingesetzt wird.

Als Poliermittel eignen sich weiter alle für Zahnpasten bekannten Putzkörper wie zum Beispiel Natriumaluminiumsilikate wie zum Beispiel Zeolith A, organische Polymere wie zum Beispiel Polymethacrylat oder Gemische dieser und der vorstehend genannten Putzkörper.

Zusammenfassend sind Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Putzkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Die angegebenen Mengen beziehen sich auf die Gesamtmenge an Putzkörpern, wobei einzelne Putzkörper vorzugsweise in engeren Mengenbereichen eingesetzt werden. Bevorzugte Mittel enthalten beispielsweise 5 bis 20 Gew.-%, vorzugsweise 8 bis 21 Gew.-%, weiter bevorzugt 9 bis 20 Gew.-% und insbesondere 10 bis 19 Gew.-% Kieselsäure(n). Weiter bevorzugte Mittel sind dadurch gekennzeichnet, dass sie 0,25 bis 2 Gew.-%, vorzugsweise 0,5 bis 1,5 Gew.-% und insbesondere 0,75 bis 1,25 Gew.-% Aluminiumoxid als weiteres Poliermittel zu der/den Kieselsäure(n) enthalten.

Als Konsistenzregler (bzw. Bindemittel) dienen zum Beispiel natürliche und/oder synthetische wasserlösliche Polymere wie Alginate, Carragheenate, Traganth, Stärke und Stärkeether, Celluloseether wie zum Beispiel Carboxymethylcellulose (Na-Salz), Hydroxyethylcellulose, Methylhydroxypropylcellulose, Guar, Akaziengum, Agar-Agar, Xanthan-Gum, Succinoglycan-Gum, Johannisbrotmehl, Pektine, wasserlösliche Carboxyvinylpolymere (zum Beispiel Carbopol®-Typen), Polyvinylalkohol, Polyvinylpyrrolidon, Polyethylenglycole, insbesondere solche mit Molekulargewichten von 1.500 - 1.000.000.

Weitere Stoffe, die sich zur Viskositätskontrolle eignen, sind zum Beispiel Schichtsilikate wie zum Beispiel Montmorillonit-Tone, kolloidale Verdickungskieselsäuren wie zum Beispiel Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol-Copolymere mit einem mittleren Molgewicht von 1.000 bis 5.000 oder eine Kombination der genannten Verbindungen in den Zahnpasten verwendet werden.

Als besonders verträglich mit den weiteren Wirkstoffen der Mund- und Zahnpflege- und-reinigungsmittel haben sich CMC und Xanthan erwiesen. Bei Einsatz dieser Verdickern ist die Wirkung besonders ausgeprägt. Besonders bevorzugte Mund- und Zahnpflege- und-reinigungsmittel sind demnach dadurch gekennzeichnet, dass sie - bezogen auf ihr Gewicht - 0,2 bis 7,5 Gew.-%, vorzugsweise 0,25 bis 5 Gew.-%, besonders bevorzugt 0,3 bis 4 Gew.-%, weiter bevorzugt 0,4 bis 3 Gew.-%, noch weiter bevorzugt 0,45 bis 2 Gew.-% und insbesondere 0,5 bis 0,8 Gew.-% Carboxymethylcellulose enthalten.

Weitere besonders bevorzugte Mund- und Zahnpflege- und -reinigungsmittel sind dadurch gekennzeichnet, dass sie - bezogen auf ihr Gewicht - 0,15 bis 5 Gew.-%, vorzugsweise 0,2 bis 2,5 Gew.-%, besonders bevorzugt 0,25 bis 1 Gew.-%, weiter bevorzugt 0,3 bis 0,75 Gew.-%, noch weiter bevorzugt 0,35 bis 0,6 Gew.-% und insbesondere 0,4 bis 0,5 Gew.-% Xanthan enthalten.

Die Mittel, insbesondere die Zahnpflege- und -reinigungsmittel, können auch Substanzen zur Erhöhung des mineralisierenden Potentials enthalten, beispielsweise calciumhaltige Substanzen wie zum Beispiel Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflege- und -reinigungsmittel enthaltenen Substanzen ab. Neben den genannten obligatorischen Komponenten können die Mund- und Zahnpflege- und-reinigungsmittel weitere, an sich bekannte Hilfs- und Zusatzstoffe enthalten. Dabei ist ein Zusatzstoff, der als Zahnpastenkomponente seit langem bekannt ist, in den Mund- und Zahnpflege- und-reinigungsmittel besonders wirksam: Calciumglycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den Mund- und Zahnpflege- und -reinigungsmittel wird Calciumglycerophosphat bevorzugt in Mengen von 0,01 - 1 Gew.-% eingesetzt. Insgesamt können die Mund- und Zahnpflege- und -reinigungsmittel übliche Hilfsmittel und Zusatzstoffe in Mengen bis zu 10 Gew.-% enthalten.

Die Mund- und Zahnpflege- und -reinigungsmittel können zum Beispiel durch Zusatz von Aromaölen und Süßungsmitteln in ihren organoleptischen Eigenschaften verbessert werden.

Als Aromaöle können alle die für Mund- und Zahnpflege- und -reinigungsmittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind zum Beispiel Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind zum Beispiel Saccharin-Natrium, Natrium-Cyclamat, Saccharose, Lactose, Maltose, Fructose.

Weitere übliche Hilfs- und Zusatzstoffe für Zahnpasten oder Zanhcremes und Mundwässer oder Mundspüllösungen sind
- Lösungsmittel und Lösungsvermittler, z.B. niedere einwertige oder mehrwertige Alkohole oder Ether, z.B. Ethanol, 1,2-Propylenglycol, Diethylenglycol oder Butyldiglycol
- Farbstoffe
- Puffersubstanzen, z.B. primäre, sekundäre oder tertiäre Alkaliphosphate oder Citronensäure-/Na-Citrat
- weitere wundheilende oder entzündungshemmende Stoffe, z.B. Allantoin, Harnstoff, Azulen, Kamillewirkstoffe, Acetylsalicylsäurederivate oder Rhodanid
- weitere Vitamine wie z.B. Ascorbinsäure, Biotin oder Tocopherol
- Mineralsalze wie z.B. Mangan-, Zink- oder Magnesiumsalze oder Natriumphosphate.

Weitere Gegenstände der Erfindung sind die Verwendung einer Verbindung, die ausgewählt ist aus der Gruppe bestehend aus Verbindungen, die unter die INCI-Bezeichnungen Polyquaternium-7 und Polyquaternium-10 fallen, und deren Mischungen zur Verringerung der Überempfindlichkeit von Zähnen, insbesondere von Zahnhälsen und die Verwendung eines Mund- und Zahnpflege- und - reinigungsmittels, enthaltend mindestens eine Verbindung, die ausgewählt ist aus der Gruppe bestehend aus Verbindungen, die unter die INCI-Bezeichnungen Polyquaternium-7 und Polyquaternium-10 fallen, und deren Mischungen zur Verringerung der Überempfindlichkeit von Zähnen, insbesondere von Zahnhälsen,

Bezüglich bevorzugter Ausführungsformen der Verwendungen gilt mutatis mutandis das zu den Mitteln Gesagte.

Die Mittel können als Zahnpasten oder Zahncremes formuliert werden. Die Mittel können in Verfahren zur Reinigung von Zähnen mittels manuell betätigter oder elektrischer Zahnbürsten verwendet werden. Im Falle elektrischer Zahnbürsten besitzen die Mittel den weiteren Vorteil, dass sie bereits in geringen Mengen wirksam sind und darüber hinaus die Mechanik des elektrischen Bürstenkopfes nicht beeinträchtigen.

Ein weiterer Gegenstand der vorliegenden Erfindung ist ein erfindungsgemäßes Mund- und Zahnpflege- und -reinigungsmittel zur Anwendung in einem Verfahren zum Verschließen eines Dentintubulus an der Oberfläche eines Zahns während der Zahnreinigung, gekennzeichnet durch die SchritteBereitstellung einer Zahnbürste mit Bürstenkopf, vorzugsweise einer Zahnbürste, deren Bürstenkopf in Bewegung versetzt werden kann;
ii. Applikation von 0,5 bis 5 g eines erfindungsgemäßen Mittels nach einem der Ansprüche 1 bis 7 auf den Bürstenkopf,
iii. 30- bis 300-sekündiges Reinigen der Zähne mit dem Mittel, vorzugsweise unter Einsatz des in Bewegung versetzten Bürstenkopfes.

Bezüglich bevorzugter Ausführungsformen der Anwendung gilt mutatis mutandis das zu den Mitteln Gesagte.

### Beispiele:

Alle Angaben in Gew.-%.

**Beispiel 1 Zahnpastaformulierung**

| | **A** | **B** | **C** | **D** | **E** | **F** |
|---|---|---|---|---|---|---|
| Sorbitol, 70%ig | 55 | 55 | 45 | 60 | 65 | 70 |
| Polyquaternium-7 | 1,0 | -- | 0,75 | 1,0 | -- | -- |
| Polyquaternium-10 | -- | 0,6 | -- | -- | 0,5 | 0,5 |
| Hydrated silica | 12 | 12 | 11 | 14 | 12,5 | 14 |
| Natriumfluorid | 0,32 | 0,32 | 0,32 | 0,32 | 0,32 | 0,32 |
| Natrium Saccharin | 0,25 | 0,25 | 0,25 | 0,25 | 0,25 | 0,25 |
| Ethanol | -- | - | -- | 1 | 2 | -- |
| Xanthan | 0,6 | 0,6 | 0,3 | 0,2 | 0,2 | 0,1 |
| Natriumlaurylsulfat | 1,5 | 1,5 | 1,5 | 1,2 | 1,2 | 1,2 |
| Cocamidopropyl Betaine | 1,3 | 1,3 | 0,6 | 0,6 | 0,6 | 0,6 |
| PEG-30 Glyceryl Stearate | 0,3 | 0,3 | 0,5 | -- | 0,5 | -- |
| PEG 400 | 3,0 | 3,0 | -- | -- | -- | -- |
| Na₂HPO₄ | 0,2 | 0,2 | 0,2 | 0,2 | 0,2 | 0,2 |
| Aroma | 1,1 | 1,1 | 0,75 | 1,5 | 0,5 | 0,75 |
| Wasser, Farbstoff und ggf. Konservierung | ad 100 | | | | | |

**Beispiel 2 Mundwasserformulierung**

| | **A** | **B** | **C** | **D** | **E** | **F** |
|---|---|---|---|---|---|---|
| Sorbitol, 70%ig | 1 | 1,5 | 2 | 2,5 | 3 | 5 |
| Polyquaternium-7 | 0,75 | 1,0 | 0,5 | -- | -- | -- |
| Polyquaternium-10 | -- | -- | -- | 1 | 1,1 | 0,6 |
| Cocamidopropyl Betaine | 0,01 | 0,01 | 0,01 | 0,01 | 0,01 | 0,01 |
| Hydrated silica | 1 | 1,5 | 2 | 2,5 | 1 | 1 |
| Natriumfluorid | 0,11 | 0,11 | 0,11 | 0,11 | 0,11 | 0,11 |
| Natrium Saccharin | 0,03 | 0,05 | 0,1 | 0,05 | 0,05 | 0,1 |
| PEG-60 Hydrogenated Castor Oil | 0,2 | 0,2 | 0,2 | 0,2 | 0,2 | 0,2 |
| Trinatriumcitrat Dihydrat | 0,1 | 0,2 | 0,3 | 0,2 | 0,1 | 0,1 |
| Citronensäure | 0,001 | 0,002 | 0,1 | 0,2 | 0,01 | 0,01 |
| Cetylpyridinium Chloride | 0,01 | 0,1 | 0,05 | 0,05 | 0,1 | 0,01 |
| Aroma | 0,5 | 0,5 | 0,5 | 0,5 | 0,5 | 0,5 |
| Wasser | ad 100 | | | | | |

Dentin mit sichtbaren Dentintubuli wurde fotografiert und von 10 geschulten Personen bewertet. Anschließend wurden die Dentinplatten entweder mit der Zahncreme A oder B behandelt und erneut bewertet. Die Anzahl der offenen/sichtbaren Dentintubuli wurde in beiden Fällen deutlich reduziert, wobei der Effekt bei der Zahncreme A noch größer war als bei Zahncreme B.

## Patentansprüche

1. Mund- und Zahnpflege- und -reinigungsmittel, enthaltend - bezogen auf sein Gewicht -
a) eine Verbindung, die unter die INCI-Bezeichnung Polyquaternium fällt, ausgewählt aus der Gruppe bestehend aus Verbindungen, die unter die INCI-Bezeichnungen Polyquaternium-7 und Polyquaternium-10 fallen, und deren Mischungen,
b) 0,01 bis 10 Gew.-% amphotere(s) Tensid(e), umfassend eine unter der INCI-Bezeichnung Cocamidopropyl Betaine bekannte Verbindung und/oder eine unter der INCI-Bezeichnung Disodium Cocoamphodiacetate bekannte Verbindung und
c) 500 bis 1600 ppm Fluorid.

2. Mund- und Zahnpflege- und -reinigungsmittel nach Anspruch 1, **dadurch gekennzeichnet, dass** es - bezogen auf sein Gewicht - 0,001 - 5 Gew.-%, vorzugsweise 0,01 - 4 Gew.-%, besonders bevorzugt 0,05 - 3 Gew.-%, außerordentlich bevorzugt 0,1 - 2 Gew.-% und insbesondere 0,5 bis 1,5 Gew.-% einer Verbindung, die unter die INCI-Bezeichnung Polyquaternium fällt, ausgewählt aus der Gruppe bestehend aus Verbindungen, die unter die INCI-Bezeichnungen Polyquaternium-7 und Polyquaternium-10 fallen, und deren Mischungen, enthält.

3. Mund- und Zahnpflege- und -reinigungsmittel nach einem der Ansprüche 1 oder 2, **dadurch gekennzeichnet, dass** das Mund- und Zahnpflege- und -reinigungsmittel weiterhin ein anionisches Tensid, insbesondere Natriumlaurylsulfat, enthält.

4. Mund- und Zahnpflege- und -reinigungsmittel nach einem der Ansprüche 1 bis 3, **dadurch gekennzeichnet, dass** das Mund- und Zahnpflege- und -reinigungsmittel weiterhin ein Poliermittel, insbesondere eine Kieselsäure, enthält.

5. Verbindung, die ausgewählt ist aus der Gruppe bestehend aus Verbindungen, die unter die INCI-Bezeichnungen Polyquaternium-7 und Polyquaternium-10 fallen, und deren Mischungen zur Verwendung bei der Verringerung der Überempfindlichkeit von Zähnen, insbesondere von Zahnhälsen.

6. Mund- und Zahnpflege- und -reinigungsmittels enthaltend - bezogen auf sein Gewicht -
a) eine Verbindung, die unter die INCI-Bezeichnung Polyquaternium fällt, ausgewählt aus der Gruppe bestehend aus Verbindungen, die unter die INCI-Bezeichnungen Polyquaternium-7 und Polyquaternium-10 fallen, und deren Mischungen
b) 0,01 bis 10 Gew.-% amphotere(s) Tensid(e), umfassend eine unter der INCI-Bezeichnung Cocamidopropyl Betaine bekannte Verbindung und/oder eine unter der INCI-Bezeichnung Disodium Cocoamphodiacetate bekannte Verbindung und
c) 500 bis 1600 ppm Fluorid zur Verwendung bei der Verringerung der Überempfindlichkeit von Zähnen, insbesondere von Zahnhälsen.

7. Mund- und Zahnpflege- und -reinigungsmittel nach einem der Ansprüche 1 bis 4 zur Anwendung in einem Verfahren zum Verschließen eines Dentintubulus an der Oberfläche eines Zahns während der Zahnreinigung, **gekennzeichnet durch** die Schritte Bereitstellung einer Zahnbürste mit Bürstenkopf, vorzugsweise einer Zahnbürste, deren Bürstenkopf in Bewegung versetzt werden kann;
ii. Applikation von 0,5 bis 5 g des Mittels auf den Bürstenkopf,
iii. 30- bis 300-sekündiges Reinigen der Zähne mit dem Mittel, vorzugsweise unter Einsatz des in Bewegung versetzten Bürstenkopfes.

## Claims

1. An oral and dental care and cleaning agent, containing, based on the weight thereof,
a) a compound which falls under the INCI name polyquaternium, selected from the group consisting of compounds which fall under the INCI names polyquaternium-7 and polyquaternium-10, and mixtures thereof,
b) 0.01 to 10 wt.% amphoteric surfactant(s), comprising a compound known under the INCI name cocamidopropyl betaine and/or a compound known under the INCI name disodium cocoamphodiacetate, and
c) 500 to 1600 ppm fluoride.

2. The oral and dental care and cleaning agent according to claim 1, **characterized in that** it contains, based on the weight thereof, 0.001 to 5 wt.%, preferably 0.01 to 4 wt.% particularly preferably 0.05 to 3 wt.%, extremely preferably 0.1 to 2 wt.% and in particular 0.5 to 1.5 wt.%, of a compound which falls under the INCI name polyquaternium, selected from the group consisting of compounds which fall under the INCI names polyquaternium-7 and polyquaternium-10, and mixtures thereof.

3. The oral and dental care and cleaning agent according to one of claims 1 or 2, **characterized in that** the oral and dental care and cleaning agent further contains an anionic surfactant, in particular sodium lauryl sulfate.

4. The oral and dental care and cleaning agent according to one of claims 1 to 3, **characterized in that** the oral and dental care and cleaning agent further contains a polishing agent, in particular a silicic acid.

5. A compound selected from the group consisting of compounds which fall under the INCI names polyquaternium-7 and polyquaternium-10, and mixtures thereof, for use in reducing hypersensitivity of teeth, in particular of tooth necks.

6. An oral and dental care and cleaning agent, containing, based on the weight thereof,
a) a compound which falls under the INCI name polyquaternium, selected from the group consisting of compounds which fall under the INCI names polyquaternium-7 and polyquaternium-10, and mixtures thereof,
b) 0.01 to 10 wt.% amphoteric surfactant(s), comprising a compound known under the INCI name cocamidopropyl betaine and/or a compound known under the INCI name disodium cocoamphodiacetate, and
c) 500 to 1600 ppm fluoride, for use in reducing hypersensitivity of teeth, in particular of tooth necks.

7. The oral and dental care and cleaning agent according to one of claims 1 to 4 for use in a method for closing a dentinal tubule on the surface of a tooth during tooth cleaning, **characterized by** the steps of providing a toothbrush which has a brush head, preferably a toothbrush of which the brush head can be set in motion;
ii. applying 0.5 to 5 g of the agent to the brush head,
ii. cleaning the teeth for 30 to 300 seconds using the agent, preferably using the brush head which is set in motion.

## Revendications

1. Produit de nettoyage et d'hygiène bucco-dentaire contenant, par rapport à son poids,
a) un composé relevant des polyquaternium selon la dénomination INCI, choisi dans le groupe constitué des composés relevant des polyquaternium-7 et polyquaternium-10 selon la dénomination INCI, et leurs mélanges,
b) de 0,01 à 10 % en poids de tensioactif(s) amphotère(s), comprenant un composé connu sous le nom de cocamidopropyl bétaïne selon la dénomination INCI et/ou un composé connu sous le nom de disodium cocoamphodiacétate selon la dénomination INCI, et
c) de 500 à 1600 ppm de fluorure.

2. Produit de nettoyage et d'hygiène bucco-dentaire selon la revendication 1, **caractérisé en ce qu'**il contient, par rapport à son poids, de 0,001 à 5 % en poids, de préférence de 0,01 à 4 % en poids, de manière particulièrement préférée de 0,05 à 3 % en poids, de manière très préférée de 0,1 à 2 % en poids, et en particulier de 0,5 à 1,5 % en poids d'un composé relevant des polyquaternium selon la dénomination INCI, choisi dans le groupe constitué par les composés relevant des polyquaternium-7 et polyquaternium-10 selon la dénomination INCI, et leurs mélanges.

3. Produit de nettoyage et d'hygiène bucco-dentaire selon l'une des revendications 1 ou 2, **caractérisé en ce que** le produit de nettoyage et d'hygiène bucco-dentaire contient en outre un tensioactif anionique, en particulier du laurylsulfate de sodium.

4. Produit de nettoyage et d'hygiène bucco-dentaire selon l'une des revendications 1 à 3, **caractérisé en ce que** le produit de nettoyage et d'hygiène bucco-dentaire contient en outre un agent de polissage, en particulier une silice.

5. Composé choisi dans le groupe constitué de composés relevant des polyquaternium-7 et polyquaternium-10 selon la dénomination INCI et leurs mélanges, destinés à être utilisés pour la réduction de l'hypersensibilité des dents, en particulier des collets dentaires.

6. Produit de nettoyage et d'hygiène bucco-dentaire contenant, par rapport à son poids,
a) un composé relevant des polyquaternium selon la dénomination INCI, choisi dans le groupe constitué des composés relevant des polyquaternium-7 et polyquaternium-10 selon la dénomination INCI, et leurs mélanges
b) de 0,01 à 10 % en poids de tensioactif(s) amphotère(s), comprenant un composé connu sous le nom de cocamidopropyl bétaïne selon la dénomination INCI et/ou un composé connu sous le nom de disodium cocoamphodiacétate selon la dénomination INCI, et
c) de 500 à 1600 ppm de fluorure destiné à être utilisé pour réduire l'hypersensibilité des dents, en particulier des collets dentaires.

7. Produit de nettoyage et d'hygiène bucco-dentaire selon l'une des revendications 1 à 4, destiné à être utilisé dans un procédé de fermeture d'un tube dentinaire à la surface d'une dent lors du nettoyage dentaire, **caractérisé par** les étapes
i. de fourniture d'une brosse à dents comportant une tête de brosse, de préférence une brosse à dents dont la tête de brosse peut être mise en mouvement ;
ii. d'application de 0,5 à 5 g du produit sur la tête de brosse,
iii. de nettoyage des dents pendant 30 à 300 secondes avec le produit, de préférence en utilisant la tête de brosse mise en mouvement.
